# EUROPEAN PATENT APPLICATION

(11) **EP 3 995 502 A1**
(43) Date of publication of application: **11.05.2022**
(21) Application number: 20835530.5
(22) Date of filing: 01.07.2020
(51) Int. Cl.: C07F 9/54, A61K 31/66

(54) **4-HYDROXYPHENYL PHOSPHONIUM SALTS WITH ANTI-PARASITIC PROPERTIES**

(30) Priority: 01.07.2019 ES 201930604
(71) Applicant: Consejo Superior de Investigaciones Científicas (CSIC), 28006 Madrid (ES); Universidad Complutense De Madrid, 28040 Madrid (ES)
(72) Inventor: DARDONVILLE, Christophe, 28006 Madrid (ES); CUETO DÍAZ, Eduardo José, 28006 Madrid (ES); GAMARRO CONDE, Francisco, 18016 Armilla (Granada) (ES); MANZANO GONZÁLEZ, José Ignacio, 18016 Armilla (Granada) (ES); PEREA MARTÍNEZ, Ana, 18016 Armilla (Granada) (ES); ALUNDA RODRÍGUEZ, José María, 28040 Madrid (ES); TORRADO DURÁN, Juan José, 28040 Madrid (ES); OLÍAS MOLERO, Ana Isabel, 28040 Madrid (ES)
(74) Representative: Pons
(86) International application number: PCT/ES2020/070421
(87) International publication number: WO 2021/001587

(57) **Abstract**

The present invention provides a new series of compounds derived from 4-hydroxyphenyl phosphonium. The invention also relates to a pharmaceutical composition comprising the compounds and to the use thereof in the treatment or prevention of diseases caused by parasites, preferably trypanosomatid parasites. In particular, these compounds have demonstrated effectiveness in vivo for the treatment of visceral leishmaniasis in a mouse model. These diseases can occur in humans and in animals.

## Description

The present invention relates to 4-hydroxyphenyl phosphonium derivatives and their potential in the treatment of diseases, both human and animal, caused by trypanosomatid parasites (leishmaniasis, trypanosomiasis). Therefore, the invention falls within the pharmaceutical and veterinary sector.

### STATE OF THE ART

Human leishmaniasis is a disease that exists worldwide, where its prevalence has reached 12 million cases. It particularly appears in tropical and subtropical regions, although it is also found in Mediterranean countries. Moreover, there is a strong incidence of infection by *Leishmania* in immunocompromised patients, especially among AIDS patients (Alvar, J. et al. Adv. Parasitol. 2006, 61, 223-74). Parasites of the genus *Leishmania* cause different forms of the disease which range from cutaneous leishmaniasis, which is milder, to visceral leishmaniasis, a much more serious form of the disease that is fatal if not treated properly. The species *Leishmania* that appears in the Mediterranean region and specifically in Spain is *L. infantum,* which causes cutaneous and visceral forms of the disease. Therapeutic options for treating leishmaniasis include pentavalent antimonial derivatives (sodium stibogluconate and meglumine antimoniate), the antibiotic amphotericin B and liposomal formulation thereof AmBisome^{®}, the antibiotic paromomycin, the alkylphosphocholine derivative miltefosine, 8-aminoquinoline sitamaquine and diamidine pentamidine (Mishra, J. et al. Curr. Med. Chem. 2007, 14, 1153-69). However, these drugs have several limitations such as their toxicity, their difficult administration, their high price and, the most worrying, the emergence of strains resistant to antimonials (Croft, S. L. et al. Clin Microbiol Rev 2006, 19, 111-26) and miltefosine (Srivastava, S. et al. Parasites & Vectors 2017,10, 49). Moreover, the treatment of the most complex forms of the disease, as well as in cases of co-infection with HIV, is not very effective.

The *in vivo* activity of tetraphenylphosphonium bromide on *L. braziliensis* (Hanson, W. L. et al. Technical reports ADA283312, 1994, 1-43) and the *in vitro* leishmanicidal activity of benzophenone-derived bisphosphonium salts (Luque-Ortega, J.R. et al. J. Med. Chem. 2010, 53, 1788-98) has been described. Moreover, phosphonium salts with activity on *Trypanosoma brucei* which are derived from benzophenone (Taladriz, A. et al. J. Med. Chem. 2012, 55, 2606-2622), 4-hydroxybenzoate (Fueyo Gonzalez, F. J. et al. J. Med. Chem. 2017, 60, 1509-1522; Ebiloma, G. U. et al. Eur. J. Med. Chem. 2018, 150, 385-402), 4-hydroxybenzaldehyde and 4-hydroxybenzoic acid (Meco-Navas, A. et al. ACS Med. Chem. Lett. 2018, 9, 923-928), triaryl benzyl phosphonium halides (US Patent 4187300A) have been described. Also, triphenyl phosphonium salts with activity on *T. cruzi* (Long, T. E. et al. Bioorg. Med. Chem. Lett. 2012, 22, 2976-2979; Cortes, L. A. et al. PLoS ONE 2015, 10, e0136852) have been described. Moreover, phosphonium salts with microbicidal activity against the fungi *Venturia inaequalis, Pyricularia oryzae, Botrytis cinerea* and *Puccinia recondita,* as well as against the protist *Plasmopara viticola* (Patent DE3332716A) have been described.

However, phosphonium salts derived from 1-(4-hydroxyphenyl)-oxo-alkane with long alkyl chain (> 14 carbons) between the benzoyl radical and the phosphonium group have not been described as leishmanicidal agents.

An advantage of the compounds described in this patent is that they are more stable against liver and plasma metabolism than the benzoate derivatives previously described. Moreover, these compounds are more selective against *Leishmania* than the aforementioned benzoates and they are effective on drug-resistant strains.

### DESCRIPTION OF THE INVENTION

The compounds described in the present invention may be useful to treat infections caused by trypanosomatid protozoa (*Leishmania* and *Trypanosoma*)*.*

Therefore, a first aspect of the present invention relates to a compound of general formula (I), pharmaceutically acceptable salt, or solvate thereof 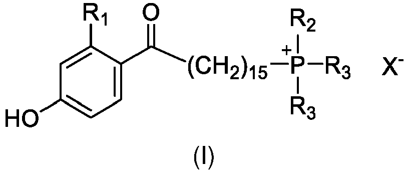 wherein:
R₁ is selected from OH and CH₃;
R₂ and R₃ are independently selected from C₁-C₆ alkyl, cycloalkyl, aryl or heterocycle;
X is a halogen.

The compounds of formula (I) can be hereinafter referred to as compounds of the invention.

The term "solvate" is intended to indicate a pharmaceutically acceptable solvate form of a specified compound that maintains the biological efficacy of said compound. Examples of solvates include compounds of the invention together with water, isopropanol, ethanol, methanol, DMSO (dimethyl sulfoxide), ethyl acetate, acetic acid or ethanolamine. The term "hydrate" is used when said solvent is water. Solvation methods are generally known in the field.

The term "pharmaceutically acceptable salt" refers to salts prepared from pharmaceutically acceptable non-toxic bases including inorganic bases and organic bases. Salts derived from inorganic bases include salts of aluminium, ammonium, calcium, copper, ferric salts, ferrous salts, lithium, magnesium, manganic salts, manganous salts, potassium, sodium, zinc and the like. Salts derived from pharmaceutically acceptable non-toxic organic bases include salts of primary, secondary and tertiary amines, substituted amines including natural substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like. When the compound of the present invention is basic, salts can be prepared from pharmaceutically acceptable non-toxic acids, including inorganic and organic acids. Such acids include acetic acid, benzenesulphonic acid, benzoic acid, camphorsulphonic acid, citric acid, ethanesulphonic acid, fumaric acid, gluconic acid, glutamic acid, hydrobromic acid, hydrochloric acid, isethionic acid, lactic acid, maleic acid, malic acid, mandelic acid, methanesulphonic acid, mucic acid, nitric acid, pamoic acid, pantothenic acid, phosphorus acid, succinic acid, sulphuric acid, tartaric acid, p-toluenesulphonic acid and the like.

Preferred examples of pharmaceutically acceptable salts include salts of ammonium, calcium, magnesium, potassium and sodium, and those formed from maleic acid, fumaric acid, benzoic acid, ascorbic acid, pamoic acid, succinic acid, hydrochloric acid, sulphuric acid, bismethylenesalicylic acid, methanesulphonic acid, ethanedisulphonic acid, propionic acid, tartaric acid, salicylic acid, citric acid, gluconic acid, aspartic acid, stearic acid, palmitic acid, itaconic acid, glycolic acid, p-aminobenzoic acid, glutamic acid, benzenesulphonic acid, cyclohexylsulfamic acid, phosphoric acid and nitric acid.

The term "alkyl" refers to, in the present invention, saturated hydrocarbon chains, linear or branched, having 1 to 6 carbon atoms, for example, methyl, ethyl, n-propyl, *i*-propyl, n-butyl, tert-butyl, sec-butyl, n-pentyl, n-hexyl, cyclohexyl. The alkyl groups can be optionally substituted by one or more substituents such as alkynyl, alkenyl, halogen, hydroxyl, alkoxyl, carboxyl, cyano, carbonyl, acyl, alkoxycarbonyl, amino, nitro or mercapto.

"Halogen" is understood in the present invention as a bromine, chlorine, iodine or fluorine atom.

In a preferred embodiment, R₁ is selected from OH and CH₃, and more preferably R₁ is OH.

In another preferred embodiment, R₂ and R₃ are aryls, and more preferably phenyl.

In another preferred embodiment, R₂ and R₃ are 1-naphthalenyl.

In another preferred embodiment, R₂ is 2-pyridyl and R₃ is phenyl.

In another preferred embodiment, the compounds of formula (I) are selected from the list comprising:
- (15-(2,4-dihydroxyphenyl)-15-oxopentadecyl)triphenylphosphonium bromide **(1) TAO99**
- (16-(4-hydroxy-2-methylphenyl)-16-oxohexadecyl)triphenylphosphonium bromide **(2) TAO100**
- (16-(4-hydroxy-2-methylphenyl)-16-oxohexadecyl)diphenyl(pyridin-2-yl)phosphonium bromide **(3) TAO101**
- (16-(2,4-dihydroxyphenyl)-16-oxohexadecyl)tri(naphthalen-1-yl)phosphonium bromide **(4) TAO118**

Another aspect of the present invention relates to a pharmaceutical composition comprising a compound of general formula (I) 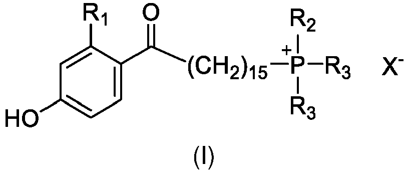 wherein:
R₁ is selected from OH and CH₃;
R₂ and R₃ are independently selected from C₁-C₆ alkyl, cycloalkyl, aryl or heterocycle;
X is a halogen.
together with a pharmaceutically acceptable carrier.

The pharmaceutically acceptable carriers that may be used in said compositions are substances known to those skilled in the art and are usually used to prepare therapeutic compositions.

The compounds and compositions of this invention can be used with other drugs to provide combination therapy. The other drugs can be part of the same composition, or they can be provided as a separate composition for administration at the same time or at a different time.

Therefore, in another preferred embodiment, the composition of the invention is characterised in that it can further comprise at least another active ingredient, preferably an anti-parasitic agent.

Another aspect of the present invention relates to the use of a compound, of general formula (I), for preparing a drug.

Another aspect of the present invention relates to the use of a compound of the invention, of general formula (I), for preparing a drug for the prevention and/or treatment of diseases caused by parasites, preferably diseases caused by protozoan parasites. These diseases occur in an animal, preferably in a mammal, including humans.

In a preferred embodiment, the diseases are diseases caused by a parasite of the genus *Leishmania* or *Trypanosoma.* And even more preferably, the parasites are of the species *L. aethiopica, L. amazonensis, L. arabica, L. archibaldi, L. aristedesi, L. braziliensis, L. chagasi (syn. L. infantum), L. colombiensis, L. deanei, L. donovani, L. enriettii, L. equatorensis, L. forattinii, L. garnhami, L. gerbili, L. guyanensis, L. herreri, L. hertigi, L. infantum, L. killicki, L. lainsoni, L. major, L. mexicana, L. naiffi, L. panamensis, L. peruviana, L. pifanoi, L. shawi, L. tarentolae, L. tropica, L. turanica, L. venezuelensis, Trypanosoma brucei, T. cruzi, T. congolense, T. equinum, T. equiperdum, T. evansi, T. vivax.*

In another preferred embodiment, the disease is selected from leishmaniasis, human African trypanosomiasis (sleeping sickness), animal trypanosomiasis, or American trypanosomiasis (Chagas disease).

Another aspect of the present invention relates to a method of treatment and/or prevention of parasitic diseases or diseases caused by parasites that comprises the administration of a therapeutically effective amount of at least one compound of the invention of formula (I), or of the pharmaceutical composition as described in the present invention, to an infected individual (in need thereof). The individual can be an animal, preferably a mammal, including humans.

In the sense used in this description, the expression "therapeutically effective amount" refers to the amount of the agent or compound capable of developing the therapeutic action determined by the pharmacological properties thereof, calculated to produce the desired effect and, in general, it will be determined, among other causes, by the very features of the compounds, as well as the age, condition of the patient, the severity of the illness or disorder, and the route and frequency of administration.

Said therapeutic composition can be prepared in solid or suspension form, in a pharmaceutically acceptable diluent. The therapeutic composition provided by this invention can be administered via any suitable route of administration, for which said composition will be formulated in the pharmaceutical form suitable for the chosen route of administration.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

### DESCRIPTION OF THE FIGURES

**Fig. 1****.** The synthetic route for preparing the derivatives of formula (I): (i) HBr 48%, 110 °C; (ii) BF₃-Et₂O, 110 °C; (iii) triphenylphosphine (for **1** and **2)** or diphenyl-2-pyridylphosphine (for **3),** CH₃CN, 80 °C, 5-18 days; (iv) tris(naphthalen-1-yl)phosphane (for **4),** DMF, 110 °C, 5 days.
**Fig. 2****.** The plasma concentration obtained after oral administration of 20 mg/kg of compound **1** in 5-8% DMSO to BALB/c mice. The values correspond to the mixture of plasmas obtained at the sampling times (n = 3).

### Examples.

Next, the invention will be illustrated by means of assays carried out by the inventors, which demonstrates the specificity and effectiveness of the compounds described in the present invention.

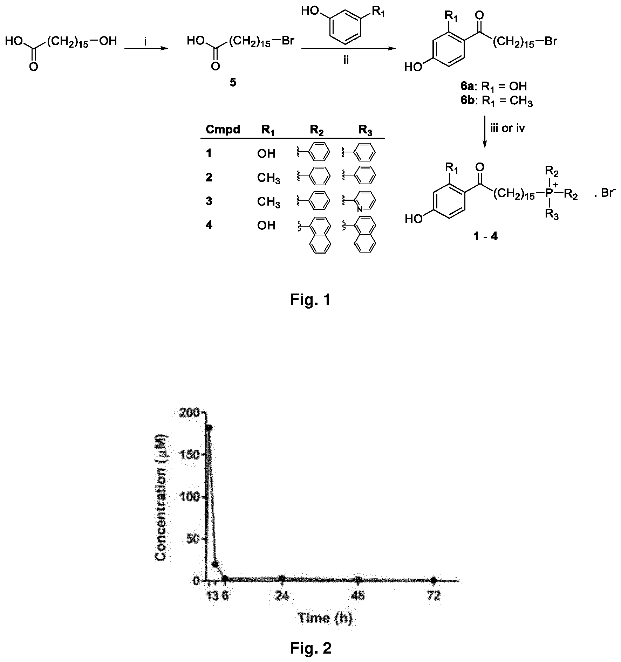

The compounds of formula (I) that are synthesised below are outlined in Figure 1.

### Example 1. Synthesis of the compounds of the invention

**16-bromohexadecanoic acid (5).** In a Kimax tube with screw cap, a solution of 16-hydroxyhexadecanoic acid (1.0 g, 3.67 mmol) in acetic acid (4 ml) and 48% aqueous HBr (4 ml) is stirred at 110 °C for 41 h. After the reaction mixture has cooled, the precipitated supernatant is collected by filtration, washed with water and dried under vacuum, obtaining 1.671 g of raw product. The compound 5 recrystallises with hexane, obtaining white crystals (1.074 g, 87%). ¹H NMR (400 MHz, CDCl₃) δ 3.34 (t, *J* = 6.9 Hz, 2H), 2.28 (t, *J* = 7.5 Hz, 2H), 1.79 (p, *J* = 7.2 Hz, 2H), 1.57 (p, J= 7.5 Hz, 2H), 1.40 - 1.12 (m, 22H) have been described. ¹³C NMR (101 MHz, CDCl₃) δ 179.9, 34.03, 34.02, 32.9, 29.62, 29.60, 29.58, 29.54, 29.44, 29.42, 29.2, 29.1, 28.8, 28.2, 24.7.

**16-bromo-1-(2,4-dihydroxyphenyl)hexadecan-1-one (6a)** In a Kimax tube (previously dried in the oven), resorcinol (33 mg, 0.3 mmol) and 16-bromohexadecanoic acid 5 (104 mg, 0.3 mmol) are added. Next, BF₃-(Et₂O)₂ (1.5 ml) is added under argon atmosphere. The tube is closed with the screw cap and the reaction mixture is stirred at 110 °C for 2 h. Next, the reaction mixture is allowed to cool and is poured onto ice. The aqueous phase is extracted with EtOAc (25 ml). The organic phase is washed with brine (5 ml), dried (MgSO₄), and evaporated, obtaining a brown oil. The product is purified by chromatography on silica (10 g) eluting with hexane/EtOAc (100/0→90/10), obtaining 6a as a white amorphous solid (58 mg, 45%). HPLC (UV) >90%. ¹H NMR (300 MHz, Chloroform-d) δ 12.81 (s, 1H), 7.59 (d, *J* = 8.9 Hz, 1H), 6.40-6.20 (m, 2H), 6.11 (brs, 1H), 3.34 (t, *J* = 7.0 Hz, 2H), 2.82 (t, *J* = 7.5 Hz, 2H), 1.78 (p, *J* = 7.1 Hz, 2H), 1.65 (p, *J* = 7.3 Hz, 2H), 1.45 - 1.06 (m, 20H) have been described. ¹³C NMR (75 MHz, CDCl₃) δ 205.6, 165.4, 162.7, 132.6, 114.0, 107.9, 103.7, 38.2, 34.3, 33.0, 29.76, 29.73, 29.67, 29.61, 29.57, 29.52, 28.9, 28.3, 25.1.

**16-bromo-1-(4-hydroxy-2-methylphenyl)hexadecan-1-one (6b).** The compound 6b is obtained by following the same procedure as for the compound 6a starting from m-cresol (30.6 mg, 0.28 mmol). The compound is purified by chromatography on silica with hexane/EtOAc (100/0 →30/1), obtaining a white amorphous solid (15 mg, 13%). HPLC (UV) > 95%. ¹H NMR (400 MHz, Chloroform-d) δ 12.36 (s, 1H), 7.57 (d, *J* = 8.3 Hz, 1H), 6.72 (d, *J* = 1.5 Hz, 1H), 6.63 (dd, *J* = 8.3, 1.5 Hz, 1H), 3.34 (t, *J* = 6.9 Hz, 2H), 2.89 - 2.84 (m, 2H), 2.28 (s, 3H), 2.25 - 2.18 (m, 2H), 1.83 - 1.74 (m, 2H), 1.66 (p, *J* = 7.4 Hz, 2H), 1.59 - 1.50 (m, 2H), 1.39 - 1.17 (m, 18H). ¹³C NMR (101 MHz, CDCl₃) δ 206.5, 162.8, 147.9, 130.0, 120.2, 118.7, 117.3, 38.4, 34.6, 34.2, 33.0, 29.74, 29.73, 29.68, 29.61, 29.59, 29.58, 29.49, 29.41, 29.29, 28.91, 28.33, 25.14, 24.86.

**(15-(2,4-dihydroxyphenyl)-15-oxopentadecyl)triphenylphosphonium bromide (1) (TAO99).** In a Kimax tube with screw cap, **6a** (75 mg, 0.18 mmol) and Ph₃P (51 mg, 0.19 mmol) in anhydrous acetonitrile (1.5 ml) are added. The reaction mixture is stirred for 18 days at 80 °C under argon atmosphere. Next, the tube is left in the refrigerator (4 °C) for several days until a white precipitate appears. The supernatant is removed and the precipitate is washed with a little amount of cold acetonitrile. The tube is centrifuged and the supernatant is removed. The same operation is repeated for washing the precipitate with Et₂O. The product 1 is isolated as a white amorphous solid (85 mg, 100%). HPLC >95%, ¹H NMR (400 MHz, Chloroform-d) δ 12.71 (s, 1H), 9.70 (s, 1H), 7.78 - 7.56 (m, 15H), 7.48 (d, *J* = 8.9 Hz, 1H), 6.55 (dd, *J* = 8.9, 2.4 Hz, 1H), 6.33 (d, *J* = 2.4 Hz, 1H), 3.41 (td, *J* = 13.1, 12.6, 7.3 Hz, 2H), 2.74 (t, *J* = 7.5 Hz, 2H), 1.60 (q, *J* = 7.4 Hz, 2H), 1.56 - 1.42 (m, 4H), 1.30 - 1.05 (m, 20H). ¹³C NMR (101 MHz, Chloroform-d) δ 205.2, 165.3, 165.0, 135.3 (d, J= 3.0 Hz), 133.6 (d, J= 9.9 Hz), 132.2, 130.7 (d, *J* = 12.5 Hz), 118.2 (d, *J* = 86.0 Hz), 112.8, 108.7, 103.4, 37.9, 30.6 (d, *J* = 15.5 Hz), 29.5, 29.4, 29.3, 29.2, 29.14, 29.11, 29.08, 25.2 (d, *J* = 1.8 Hz), 22.8 (d, *J* = 50.3 Hz), 22.7 (d, *J* = 4.5 Hz). HRMS (ESI⁺) *m*/*z* 609.3482 (C₄₀H₅₀PO₃ requires: 609.3498).

**(16-(4-hydroxy-2-methylphenyl)-16-oxohexadecyl)triphenylphosphonium bromide (2) (TAO100).** In a Kimax tube, 6b (6.9 mg, 0.016 mmol), Ph₃P (4.2 mg, 0.016 mmol) and 0.6 ml of anhydrous DMF are added under argon atmosphere. The tube is closed with the screw cap and the reaction mixture is stirred at 80 °C for 5 days. The solvent is evaporated and the product dissolved in MeOH is isolated by precipitation with Et₂O/hexane at 4 °C overnight. The supernatant is removed and the precipitate is washed successively with Et₂O and hexane. Off-white amorphous solid (7.6 mg, 69%). HPLC > 95%. ¹H NMR (400 MHz, Chloroform-d) δ 12.36 (s, 1H), 7.84 - 7.74 (m, 6H), 7.72 (td, *J* = 7.3, 1.8 Hz, 3H), 7.63 (td, *J* = 7.7, 3.5 Hz, 6H), 7.57 (d, *J* = 8.2 Hz, 1H), 6.71 (d, *J* = 1.7 Hz, 1H), 6.63 (dd, *J* = 8.2, 1.7 Hz, 1H), 3.76 (dt, *J* = 13.2, 7.4 Hz, 2H), 2.87 (t, *J* = 7.5 Hz, 2H), 2.27 (s, 3H), 1.66 (p, *J* = 7.4 Hz, 2H), 1.60 - 1.50 (m, 2H), 1.38 - 1.07 (m, 22H). ¹³C NMR (101 MHz, Chloroform-d) δ 206.6, 162.8, 147.9, 135.1 (d, *J* = 3.0 Hz), 133.9 (d, *J* = 10.0 Hz), 130.6 (d, *J* = 12.5 Hz), 130.1, 120.3, 118.7 (d, *J* = 85.6 Hz), 118.6, 117.3, 38.4, 30.6 (d, *J* = 15.5 Hz), 29.71, 29.69, 29.67, 29.64, 29.57, 29.54, 29.45, 29.41, 29.3, 24.8, 23.0 (d, *J* = 49.3 Hz), 22.8 (d, *J* = 4.6 Hz), 22.1. HRMS (ESI⁺) *m*/*z* 607.3709 (C₄₁H₅₂PO₂ requires: 607.3705).

**(16-(4-hydroxy-2-methylphenyl)-16-oxohexadecyl)diphenyl(pyridin-2-yl)phosphonium bromide (3) TAO101.** The same procedure is used for the synthesis of the compound **2.** A mixture of **6b** (5.7 mg, 0,014 mmol) and Ph₃P (3.7 mg, 0.014 mmol) in anhydrous acetonitrile (0.6 ml) is stirred at 80 °C for 5 days under argon atmosphere. The solvent is evaporated and the product dissolved in MeOH is isolated by precipitation with Et₂O at 4 °C overnight. The supernatant is removed and the precipitate is washed successively with Et₂O and hexane. Brown amorphous solid (4.6 mg, 48%). HPLC > 95%. ¹H NMR (400 MHz, Chloroform-d) δ 12.36 (s, 1H), 8.81 (dt, *J* = 4.7, 1.5 Hz, 1H), 8.42 (ddt, *J* = 7.9, 5.9, 1.1 Hz, 1H), 8.09 (tdd, *J* = 7.9, 5.1, 1.8 Hz, 1H), 7.87 - 7.78 (m, 4H), 7.77 - 7.70 (m, 2H), 7.62 (tdd, *J* = 9.1, 4.5, 3.2 Hz, 5H), 7.57 (d, *J* = 8.0 Hz, 1H), 6.71 (dd, J= 1.6, 0.9 Hz, 1H), 6.63 (dd, J= 8.0, 1.6 Hz, 1H), 3.75 - 3.62 (m, 2H), 2.92 - 2.82 (m, 2H), 1.72 - 1.46 (m, 4H), 1.37 - 1.07 (m, 22H). ¹³C NMR (101 MHz, Chloroform-d) δ 206.6, 162.8, 151.9 (d, *J* = 19.1 Hz), 147.9, 145.4, 144.2, 138.8 (d, *J* = 10.3 Hz), 135.1 (d, *J* = 3.0 Hz), 134.1 (d, *J* = 9.6 Hz), 132.5 (d, *J* = 24.5 Hz), 130.5 (d, *J* = 12.6 Hz), 130.1, 128.1 (d, *J* = 3.5 Hz), 120.3, 118.6, 117.9 (d, *J* = 85.4 Hz), 117.3, 38.4, 30.6 (d, *J* = 15.2 Hz), 29.72, 29.70, 29.68, 29.63, 29.58, 29.56, 29.46, 29.32, 29.28, 24.8, 22.62, 22.59 (d, *J* = 13.6 Hz), 22.1, 22.0. HRMS (ESI⁺) *m*/*z* 608.3666 (C₄₀H₅₁ PNO₂ requires: 608.3657).

**(16-(2,4-dihydroxyphenyl)-16-oxohexadecyl)tri(naphthalen-1-yl)phosphonium bromide (4) (TAO118).** In a Kimax tube, **6a** (53.6 mg, 0.13 mmol), tris(naphthalen-1-yl)phosphane (67.0 mg, 0.16 mmol) and 2.0 ml of anhydrous DMF are added under argon atmosphere. The tube is closed with the screw cap and the reaction mixture is stirred at 110 °C for 5 days. The solvent is evaporated and the crude solid obtained is purified by chromatography on silica with hexane/EtOAc (0→20%), obtaining a mixture of **2** with tri(naphthalen-1-yl)phosphine oxide. The latter is removed by preparative thin layer chromatography (PTLC) eluting with EtOAc/MeOH (8/2). The pure compound **4** is an off-white solid (7 mg, 6.4%). HPLC > 95%. ¹H NMR (500 MHz, Methanol-*d*₄) δ 8.43 (dd, *J* = 8.5, 1.5 Hz, 3H), 8.20 - 8.11 (m, 6H), 7.97 (d, *J* = 8.6 Hz, 3H), 7.78 - 7.61 (m, 7H), 7.54 - 7.47 (m, 3H), 6.33 (ddd, *J* = 8.8, 2.4, 0.6 Hz, 1H), 6.20 (d, *J* = 2.4 Hz, 1H), 4.00 (br, 2H), 2.88 (t, *J* = 7.4 Hz, 2H), 1.68 (p, *J* = 7.4 Hz, 2H), 1.63 - 0.95 (m, 22H). HRMS (ESI⁺) m/z 759.3981 (C₅₂H₅₆O₃P requires 759.3967).

### Example 2. In vitro assays of the leishmanicidal activity of the compounds of the invention

The compounds were evaluated *in vitro* against promastigote and intracellular amastigote forms of the parasite *L. donovani* (strain HU3) following the MTT colorimetric method described previously (Kennedy ML, et al. J Med Chem 2001, 44, 4668-4676). Moreover, their cytotoxicity on human THP-1 cells was evaluated and the selectivity index (SI) of each compound was calculated (Table 1).

The compounds object of this patent show activities on promastigotes and intracellular amastigotes of *L. donovani* in the submicromolar range (0.07 - 0.28 µM), being two and ten times more potent, respectively, than the reference drugs amphotericin B and miltefosine in this assay. Moreover, the compounds show selectivity towards *Leishmania* with selectivity indices (SI) > 20 against human THP-1 cells.

**Table 1.^{a}**

| Ref. | Cmpd | EC₅₀ (µM) | | | SI*^{b}* |
|---|---|---|---|---|---|
| | | *L. donovani* promastigotes | *L. donovani* intracellular amastigotes | THP-1 cells | |
| TAO99 | **1** | 0.07 ± 0.02 | 0.09 ± 0.02 | 2.81 ± 0.35 | 31.2 |
| TAO100 | **2** | 0.09 ± 0.01 | 0.10 ± 0.01 | 2.45 ± 0.41 | 24.5 |
| TAO101 | **3** | 0.28 ± 0.05 | 0.10 ± 0.02 | 2.11 ± 0.12 | 21.1 |
| TAO118*^{d}* | **4** | 3.54 ± 0.36 | 1.25 ± 0.22 | 12.85 ± 2.30 | 10.3 |
| | AmB*^{e}* | 0.13 ± 0.02 | 0.24 | 27.85 | 116.0 |
| | MIL*^{f}* | 6.51 ± 0.35 | 0.91 ± 0.05 | 26.25 ± 2.58 | 28.8 |

*^{a}* Parasites and THP-1 cells were cultured in the presence of increasing concentrations of the compounds. The data represent the mean ± standard deviation of 3 independent experiments. *^{b}* SI = selectivity index (EC₅₀ THP-1 / EC₅₀ parasite (amastigote form)). *^{c}* not assayed. *^{d}* Sensitivity data obtained in *L. infantum* LEM3049. *^{e}*Amphotericin B. *^{f}*Miltefosine.

### Example 3. Activity of compound 1 against drug-resistant strains of L. infantum.

No significant differences in sensitivity to compound **1** were found between the drug-sensitive reference line (LEM3049) and the strains LEM 3323 (resistant to Sb^{III}), LEM 5159 (resistant to miltefosine) and LEM 2126 (resistant to miltefosine and paramomycin) (Table 2).

**Table 2. Leishmanicidal activity of compound 1 on promastigote and intracellular amastigote forms of clinical isolates of L. infantum^{a}**

| *L. infantum* | EC₅₀ (µM) | | | |
|---|---|---|---|---|
| | Promastigotes | | Intracellular amastigotes | |
| | Cmpd 1 | AmB | Cmpd 1 | AmB |
| **LEM3049** | 0.08 ± 0.03 | 0.06 ± 0.02 | 0.12 ± 0.06 | 0.17 ± 0.02 |
| **LEM3323** | 0.10 ± 0.02 | 0.07 ± 0.03 | 0.15 ± 0.07 | 0.18 ± 0.02 |
| **LEM5159** | 0.11 ± 0.02 | 0.05 ± 0.01 | 0.14 ± 0.02 | 0.16 ± 0.03 |
| **LLM2126** | 0.12 ± 0.03 | 0.10 ± 0.02 | 0.14 ± 0.01 | 0.16 ± 0.01 |

^{a}Clinical isolates of *L. infantum* used: LEM 3049 as a reference line; LEM 3323, resistant to Sb^{III}; LEM 5159, resistant to miltefosine (MIL) and LEM 2126, resistant to MIL and paromomycin. Amphotericin B (AmB) was used as a reference drug. The data represent the mean ± standard deviation of 3 independent experiments.

### Example 4. In vivo efficacy in a mouse model of visceral leishmaniasis

The compound is slightly soluble in water, but it solubilises in 5-8% DMSO/water, 20% EtOH/water or can be formulated as a colloidal suspension in 5% γ-cyclodextrin in water for oral administration (Table 3).

**Table 3. Thermodynamic solubility of compound 1 at 24 h**

| Formulation | Solubility | |
|---|---|---|
| | µM | mg/l |
| 20% ethanol in MilliQ water | 150 ± 10 | 100 ± 10 |
| 5% by weight of γ-cyclodextrin in MilliQ water | 750 ± 10 | 520 ± 10 |

Based on pharmacokinetic data, stability and absorption, compound **1** was administered orally in a BALB/c mouse model of visceral leishmaniasis. Three groups of mice were established: (i) G1, uninfected mice + 30 µg of compound **1** per mouse for 4 days (once a day); (ii) G2, infected mice + γ-cyclodextrin in MilliQ water, and (iii) G3, infected mice + compound **1** following the same schedule described for G1.

The parasitic load in the target organs (liver and spleen) was determined after 48 h of the end of treatment with compound **1.** As such, a reduction in the load of 98.9% in the spleen and 95.3% in the liver was observed when comparing the groups of mice infected and treated with the compound **1** with those infected but not treated (Table 4).

**Table 4. In vivo activity of compound 1 in the mouse model (BALB/c) of visceral leishmaniasis using Leishmania infantum.**

| **Study group** | **Parasitic load** | |
|---|---|---|
| | **Liver** | **Spleen** |
| G1 (Uninfected + compound 1 30 µg p.o.^{b} for 4 days) | - | - |
| G2 (Infected + carrier [5% γ-cyclodextrin] p.o. for 4 days) | 4.92 ± 1.21^{a} | 607.25 ± 176.96 |
| G3 (Infected + compound 1 30 µg p.o. for 4 days) | 0.18 ± 0.07 (95.3)^{c} | 6.47 ± 1.77 (98.9) |

^{a}Mean number of amastigotes per milligram of tissue. ^{b}p.o., orally. ^{c} The percentage of reduction compared to G2 is reflected in the value in parentheses.

The observed reduction of parasitic load in the target organs after treatment with compound 1 at a low dose (1.5 mg/kg once a day for 4 days) in our mouse model is similar to that obtained with miltefosine with much higher doses (i.e., 40 mg/kg once a day for 5 days) in the case of this drug (Sebastián-Pérez, V. et al. Antimicrob. Agents Chemother. 2018, 62, e00603-00618). Therefore, compound **1** is more potent *in vivo* than the oral reference drug miltefosine.

### Example 5. Pharmacokinetics, bioavailability and liver metabolism

The bioavailability of 1 after oral administration of 20 mg/kg of compound in 5-8% DMSO to BALB/c mice is very high (Cmax = 182 µM) and very fast (Tmax = 1 h) (Table 5 and Figure 2).

The liver metabolism of compound **1** is species dependent. The microsomal stability of **1** towards metabolism by cytochrome P450 (Phase I metabolism) and uridine glucuronosyltransferase (UGT) (Phase II metabolism) in the presence of NADPH and UDPGA was studied. Compound **1** was quickly metabolised by mouse liver microsomes (CD-1) (intrinsic high clearance) with half-life < 30 min, which confirms the pharmacokinetic data observed *in vivo.* By contrast, **1** was slowly metabolised by human liver microsomes (low to moderate intrinsic clearance) with t_{1/2}> 2 h, and very slowly metabolised by the S9 fraction of the human liver within 2 h of the reaction (Table 5). In comparison, high intrinsic clearance of the control drug diclofenac was observed by human fractions under the same conditions (t_{1/2} <30 min).

**Table 5. Pharmacokinetics and liver metabolism**

| Pharmacokinetics of compound 1 | |
|---|---|
| Tₘₐₓ | 1 h |
| Cmax | 182 µM |
| t_{1/2} | 1 h |
| First phase (< 6 h) | 23 h |
| Second phase (6-72 h) | |

| Liver metabolism of compound 1 | |
|---|---|
| t_{1/2} mouse (microsomes, phase I/II) | < 30 min |
| t_{1/2} human (microsomes, phase I) | > 2 h |
| t_{1/2} human (S9 fraction, phase II) | > 2 h |

### Example 6. In vitro assays of the trypanocidal activity of the compounds of the invention.

The compounds of the invention show activities in the nanomolar range against the sensitive strain (s427) and the drug-resistant strain B48 of the parasite *Trypanosoma brucei* (Table 6).

**Table 6.^{a}**

| Ref. | Cmpd | EC₅₀ (µM) | | |
|---|---|---|---|---|
| | | *T. brucei^{b}* | *T. brucei B48^{c}* | RF*^{d}* |
| TAO99 | **1** | 0,032 ± 0.0004 | 0,040 ± 0,027 | 1.27 |
| TAO100 | **2** | 0,043 ± 0.0035 | 0,044 ± 0,007 | 1.01 |
| TAO101 | **3** | 0,107 ± 0,006 | 0,096 ± 0.01 | 0.90 |
| | Pent*^{e}* | 0.0033 ± 0.0006 | 0.55 ± 0.03 | 168 |
| | Dimin*^{f}* | 0,101 ± 0,005 | 1.33 ± 0.14 | 13.1 |

*^{a}* The data represent the mean ± standard deviation of 3 independent experiments. *^{b}* Strain s427 of *T. b. brucei. ^{c}* Strain B48 of *T. b. brucei* resistant to pentamidine and diminazene. *^{d}* RF = resistance factor (EC₅₀ *T. b.* B48 / EC₅₀ *T. b*. s427). *^{e}* Pentamidine. *^{f}* Diminazene.

## Claims

1. A compound of general formula (I), pharmaceutically acceptable salt, or solvate thereof 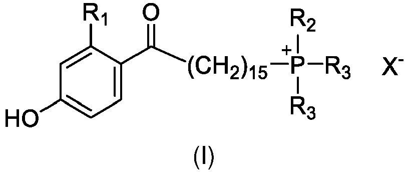 wherein:
R₁ is selected from OH and CH₃;
R₂ and R₃ are independently selected from C₁-C₆ alkyl, cycloalkyl, aryl or heterocycle;
X is a halogen.

2. The compound according to claim 1, wherein R₁ is OH.

3. The compound according to any of claims 1-2, wherein R₂ and R₃ are aryls, and more preferably phenyl.

4. The compound according to any of claims 1-2, wherein R₂ and R₃ are 1-naphthalenyl.

5. The compound according to any of claims 1-2, wherein R₂ is 2-pyridyl and R₃ is phenyl.

6. The compound according to claim 1 selected from the list comprising:
- (15-(2,4-dihydroxyphenyl)-15-oxopentadecyl)triphenylphosphonium bromide **(1) TAO99**
- (16-(4-hydroxy-2-methylphenyl)-16-oxohexadecyl)triphenylphosphonium bromide **(2) TAO100**
- (16-(4-hydroxy-2-methylphenyl)-16-oxohexadecyl)diphenyl(pyridin-2-yl)phosphonium bromide **(3) TAO101**
- (16-(2,4-dihydroxyphenyl)-16-oxohexadecyl)tri(naphthalen-1-yl)phosphonium bromide **(4) (TAO118).**

7. A pharmaceutical composition comprising a compound of general formula (I) described according to any of claims 1 to 6, together with a pharmaceutically acceptable carrier.

8. The composition according to claim 7, further comprising another anti-parasitic active ingredient.

9. The compound of general formula (I) described according to any of claims 1 to 6 for use as a drug.

10. The compound of general formula (I) for use as a drug according to claim 9, wherein the drug is for the prevention and/or treatment of diseases caused by parasites.

11. The compound of general formula (I) for use as a drug according to claim 10, wherein the disease is caused by protozoan parasites.

12. The compound of general formula (I) for use as a drug according to claim 11, wherein the protozoan parasites are of the genus *Leishmania* or *Trypanosoma.*

13. The compound of general formula (I) for use as a drug according to claim 12, wherein the parasites are of the species *L. aethiopica, L. amazonensis, L. arabica, L. archibaldi, L. aristedesi, L. braziliensis, L. chagasi (syn. L. infantum), L. colombiensis, L. deanei, L. donovani, L. enriettii, L. equatorensis, L. forattinii, L. garnhami, L. gerbili, L. guyanensis, L. herreri, L. hertigi, L. infantum, L. killicki, L. lainsoni, L. major, L. mexicana, L. naiffi, L. panamensis, L. peruviana, L. pifanoi, L. shawi, L. tarentolae, L. tropica, L. turanica, L. venezuelensis, Trypanosoma brucei, T. cruzi, T. congolense, T. equinum, T. equiperdum, T. evansi, T. vivax.*

14. The compound of general formula (I) for use as a drug according to any of claims 9 to 13, wherein the diseases occur in animals, preferably in a mammal.

15. The compound of general formula (I) for use as a drug according to any of claims 9 to 14, wherein the disease is selected from leishmaniasis, human African trypanosomiasis (sleeping sickness), animal trypanosomiasis, American trypanosomiasis (Chagas disease).
